# EUROPEAN PATENT SPECIFICATION

(11) **EP 0 580 629 B1**
(45) Date of publication and mention of the grant of the patent: **24.09.1997**
(21) Application number: 92907361.7
(22) Date of filing: 07.04.1992
(51) Int. Cl.: A61M 16/06

(54) **EQUIPMENT AND METHOD FOR GAS EXTRACTION IN GENERAL ANAESTHESIA**
VORRICHTUNG ZUM ABSAUGEN VON GAS
EQUIPEMENT ET METHODE D'EXTRACTION DE GAZ LORS D'UNE ANESTHESIE GENERALE

(30) Priority: 12.04.1991 GB 91078063
(43) Date of publication of application: 02.02.1994
(73) Proprietor: N.J ROCHESTER LIMITED, Beaconsfield, Buckinghamshire HP9 1QL (GB)
(72) Inventor: ROCHESTER, Noel Joseph, Gerrards Cross, Buckinghamshire SL9 7HS (GB)
(74) Representative: Coles, Graham Frederick
(86) International application number: GB9200621
(87) International publication number: WO9218189

(56) References cited:
- US-A- 2 418 473
- US-A- 3 877 691
- US-A- 4 082 092

## Description

This invention relates to gas-extraction equipments and methods of gas-extraction of the kind in which collection of gas to be extracted is via an open mouth of a transparent hood that extends from a support arm, the collected gas is drawn off from the hood via a port in the hood, and the hood is adjustable in orientation with respect to the arm.

Gas-extraction equipment and a method of gas-extraction of the above-specified kind are known from US-A-4082092. The known equipment and method involve a hood of part-cylindrical form which, as a modification of a flattened, shallow shield, is mounted by a universal joint at the top of the hood to extend downwardly from the end of a support boom or arm. Gas collected in the hood is drawn off into a vacuum line via a port in the upper part of the cylindrical surface of the hood.

The part-cylindrical hood of US-A-4082092 is described as useful for extracting objectionable gases from areas of incision in surgery and also from welding sites, but reference is also made to its use in the context of general anaesthesia. In general anaesthesia there is danger of the anaesthetist and others attending the human or animal patient, being themselves affected by the gas administered. This danger can arise from leakage occurring from the mask or other device used to administer the gas, and also from residual gas expired by the patient. There is accordingly a need to collect the waste gas and dispose of it effectively, but the equipment and method used in this context must not significantly obstruct view of, and access to, the region covered by the hood.

Although the equipment and the method described in US-A-4082092 using the part-cylindrical hood, is capable of reducing some of the danger of gas leakage, view of the region covered by the hood is obstructed to a significant extent by the connections of the hood to the arm and the vacuum line, and is in any case limited by the configuration of the hood itself. If the flattened, shallow shield were used, undistorted view of the patient would be improved within the limited, narrow angle of the flattened part, but the collection of waste gas would be very much less effective.

It is one of the objects of the present invention to provide a form of gas-extraction equipment and method of gas-extraction of the said above-specified kind, that may be used to overcome the disadvantages of the known gas-extraction equipment and method, especially, though not exclusively, in the context of general anaesthesia.

According to the present invention, gas-extraction equipment and a method of gas-extraction of the said above-specified kind, are characterised in that the hood is a transparent shell that is substantially hemispherically domed, that the port is located low down on the domed shell adjacent the open mouth, and that the shell is mounted on the arm at the location of the port such that except at that location, view through the hood into the region encompassed by the open mouth is substantially unobstructed and undistorted from all angles over and around the domed shell.

The use of a hood in the form of a transparent shell that is substantially hemispherically domed, has been found to be of especial advantage in the context of surgery and other procedures where anaesthetic gas is involved; it may in this regard find application generally in dental surgeries, in anaesthetic administration and operating rooms, and in post-surgery recovery areas. More particularly, the use of a substantially hemispherically-domed shell with its gas-extraction port and arm-mounting low down adjacent the open mouth, enables view of the patient through the hood to be undistorted and uninterrupted over a wide field whilst still enabling efficient collection of waste gas.

The amount of waste gas to be drawn off during general anaesthesia increases rapidly to a peak during exhalation by the patient, and there is normally the danger with known forms of hood that some of this gas will spill out from beneath the hood unless the rate of draw off is high. The large reservoir volume of the substantially-hemispherical hood used according to the present invention, allows for accumulation of the exhaled gas in a way that effectively smooths out the demands on the equipment. This in general enables a lower, constant draw-off rate to be utilised than otherwise would be required to avoid a material degree of spillage of the waste gas.

US-A-3877691 describes a form of shield for use during anaesthesia, in which two arcuate or partially-spherical panel sections are mounted one within the other. Openings through the inner section enable waste gas to be drawn into a space between the sections for extraction, but because of the limited enclosed-volume within the arcuate shield as a whole, a very high draw-off rate will be necessary to avoid significant spillage. Also, although the panel sections are both transparent and partially spherical, the view through them and the intervening space of the double-thickness wall they form, will inevitably be less than clear and in any case will be subject to distortion where the openings through the inner section occur. All these disadvantages are to a very substantial extent avoided using the equipment and method of the present invention.

With the equipment according to an embodiment of the present invention, a flange for mounting the hood to the arm may encircle the port within a flattened portion of the shell, and the mounting of the shell to the arm may involves a joint located adjacent the shell. The joint may enable the hood to be adjusted in tilt about an axis transverse to the arm, and in rotation about a longitudinal axis of the arm. This joint is preferably balanced such that the hood remains in the orientation to which it is adjusted and only light hand-pressure is required for the adjustment.

The path for gas extracted from the hood via the port in the equipment of an embodiment of the invention, may extend internally of the arm, and the arm may be carried by a telescopic column for height adjustment of the hood. For manoeuvrability, the column may be trolley-mounted, or may be mounted on a track that enables movement laterally of the column; the track may be ceiling mounted.

The rim of the substantially-hemispherical shell around the open mouth may be relieved throughout a substantial part of its periphery away from the port in order to facilitate access under the hood.

Examples of gas-extraction equipment and methods, in accordance with the present invention, will now be described with reference to the accompanying drawings, in which:
Figures 1 and 2 are side and front elevations respectively, of portable gas-extraction equipment according to an embodiment of the invention;
Figures 3 to 5 are respectively a side elevation, a rear elevation and a plan of the transparent domed-hood used in the gas-extraction equipment of Figures 1 and 2;
Figures 6 and 7 are front and side views, respectively of a swivel-joint mounting used for the transparent domed-hood in the gas-extraction equipment of Figures 1 and 2;
Figure 8 illustrates use of the gas-extraction equipment in a surgical operation; and
Figures 9 and 10 illustrate an alternative, ceiling-mounted form of gas-extraction equipment according to an embodiment of the invention.

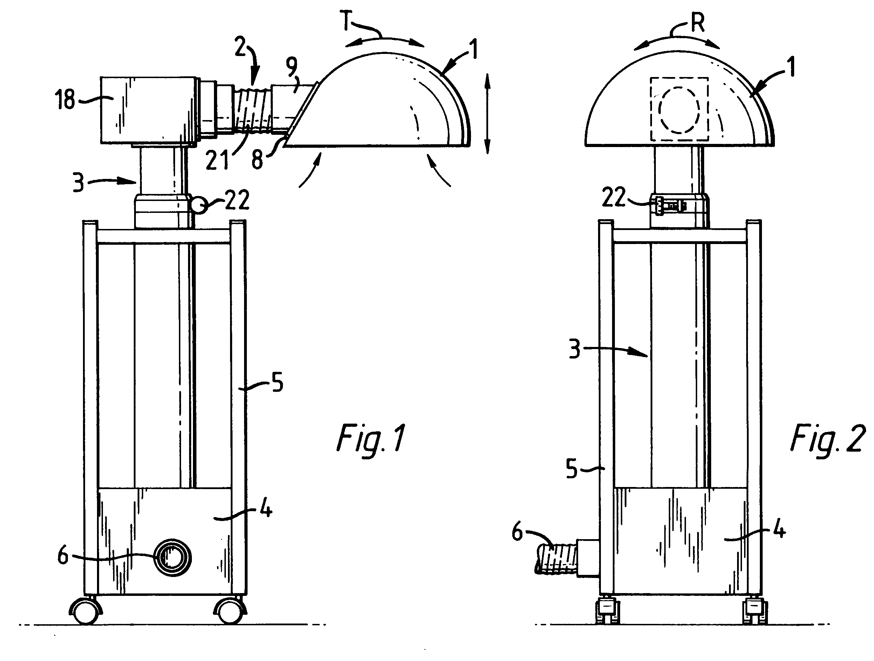

The gas-extraction equipment to be described with reference to Figures 1 and 2 may find application in dental surgery, and will be described in this context. However, the invention is not in general limited to this, and indeed the equipment to be described may be used in other contexts whether surgery is to the head or other parts of the body of a human or animal patient. The likelihood of those attending the patient being affected by the anaesthetic gas is intensified where dental surgery or other surgery to the head is being carried out, by virtue of the essential proximity of the surgeon as well as the anaesthetist, to the region of administration of the gas and exhalation by the patient.

The gas-extraction equipment to be described is adapted to reduce that likelihood without obstructing view of the site of surgery or unduly limiting access to it.

Referring to Figures 1 and 2, the gas-extraction equipment involves a gas-collection hood 1 that extends from a generally-horizontal tubular arm 2. The arm 2 projects from a tubular column 3 which is mounted to extend vertically upwards from an electrically-driven pump or fan unit 4 carried by a trolley 5. The fan unit 4 draws air from the collection hood 1 along the arm 2 and down the column 3 to exhaust it remotely through outlet ducting 6.

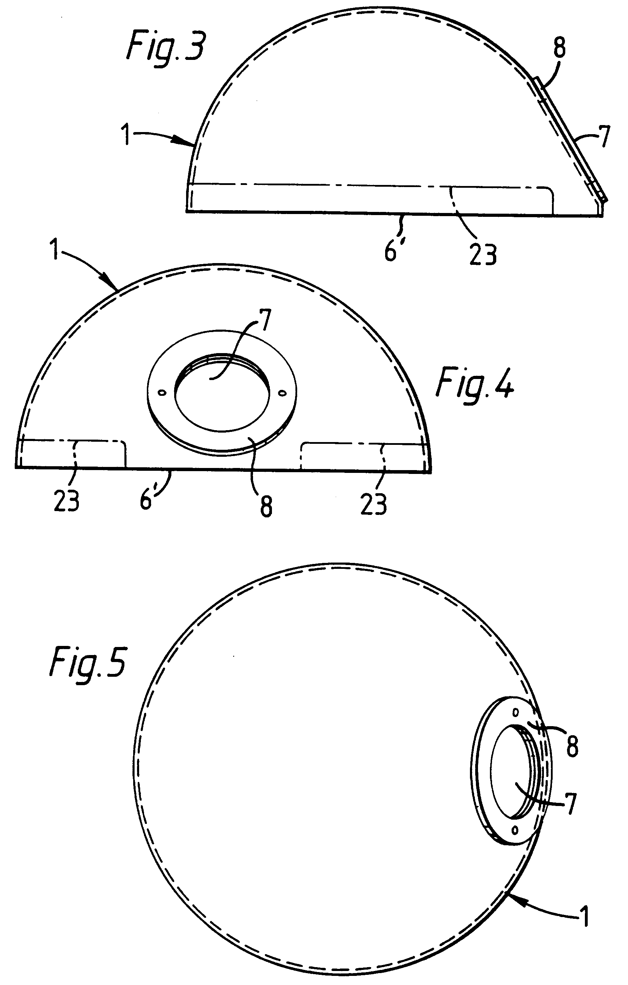

The hood 1, as shown in Figures 3 to 5, is of a clear acrylic resin having a thin-wall, but substantially rigid, shell construction with a circular, open mouth 6'. The shell, which may, for example, be of polymethyl methacrylate and have a wall-thickness of 4 mm, is of a domed form that is generally hemispherical except rearwardly where it is flattened and has a circular outlet opening or port 7. The port 7 is located low in the hood 1 close to the open mouth 6', and has an encircling annular flange 8 by which the hood 1 is releasably coupled to the arm 2.

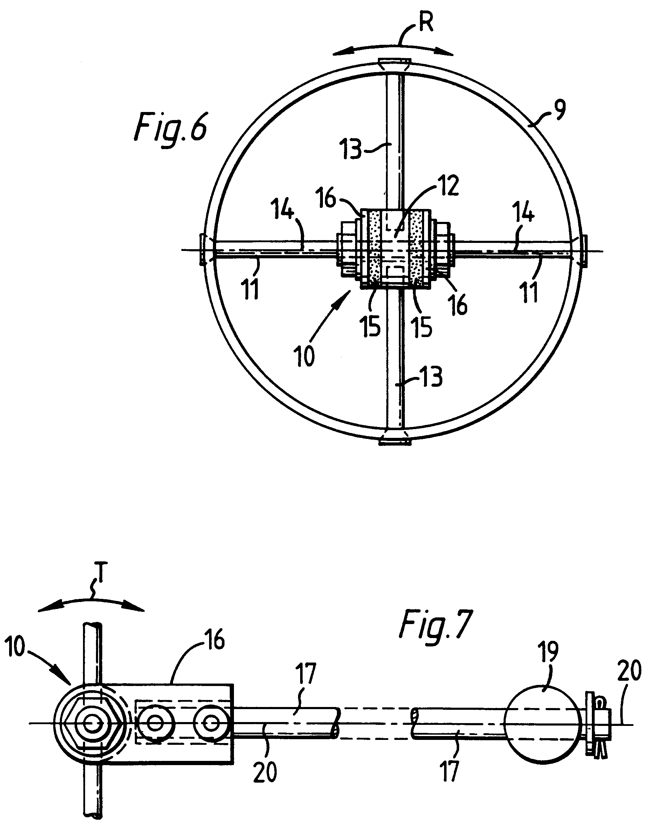

The coupling between the hood 1 and the arm 2 is made via a short length of substantially-rigid tubing 9 that is clamped to the flange 8 and houses a swivel joint 10 close to the hood 1. The joint 10, which is illustrated only in Figures 6 and 7, allows the orientation of the tubing 9 and its attached hood 1, to be adjusted angularly in tilt (indicated by arrows T in Figures 1 and 7), as well as in rotation (indicated by arrows R in Figures 2 and 6).

Referring to Figures 6 and 7, the swivel joint 10 is mounted centrally within the tubing 9 on a diametrically-extending shaft 11. A central bush 12 of the joint 10 is rotatable on the shaft 11 and is coupled to the tubing 9 at right angles to the shaft 11 by support arms 13 so as to enable the tubing 9 to tilt about the shaft-axis 14. Friction pads 15 are clamped to either side of the bush 12 by plates 16 that are carried by the shaft 11, so as to restrain the bush 12, and with it the tubing 9 and attached hood 1, from tilting (T) about the axis 14 except under hand pressure.

The plates 16 are secured to one end of a rod 17 that extends centrally the length of the arm 2. The rod 17 supports the assembly of the shaft 11, swivel joint 10, tubing 9 and attached hood 1, from within a unit 18 (Figure 1) that tops the column 3. Within the unit 18, the rod 17 extends through a transversely-mounted arm 19 and is retained there with freedom to rotate about its own longitudinal axis 20. The freedom of the shaft 17 for rotation enables the assembly including the hood 1 to be rotated about the axis 20, so that the orientation of the hood 1 is adjustable in rotation (R) as well as in tilt (T). The balance of the assembly is such as to ensure that the hood 1 remains in the orientation to which it is adjusted by hand, and that only light hand pressure is required to be exerted on the hood 1 to change that orientation in tilt (T) and/or rotation (R).

The rod 17 is enclosed throughout the length of the arm 2 from the tubing 9 to the unit 18, by flexible tubing 21 so that gas drawn through the hood 1 is passed to the column 3 without obstruction, for any hood-orientation. The unit 18 houses a rotatable joint to allow the tubing 9 to rotate about the rod 17 when the orientation of the hood 1 is adjusted, as well as bearings to enable the arm 2 to be swung round to any convenient location about the vertical column 3. Frictional restraint keeps the arm 2 from moving too easily from wherever it is placed in this respect, yet allows for displacement by easy touch of the hand. (Instead of the unit 18 being rotatable relative to the column 3, the column 3 may itself be mounted for rotation about the vertical, so that adjustment of the angular position of the arm 2 in the horizontal is achieved by rotation of the unit 18 and column 3 together as one, relative to the trolley 5. Furthermore, instead of providing for the rod 17 to rotate for adjustment of the hood 1 in rotation (R), the swivel joint 10 may be replaced by a universal joint mounted on a fixed rod.)

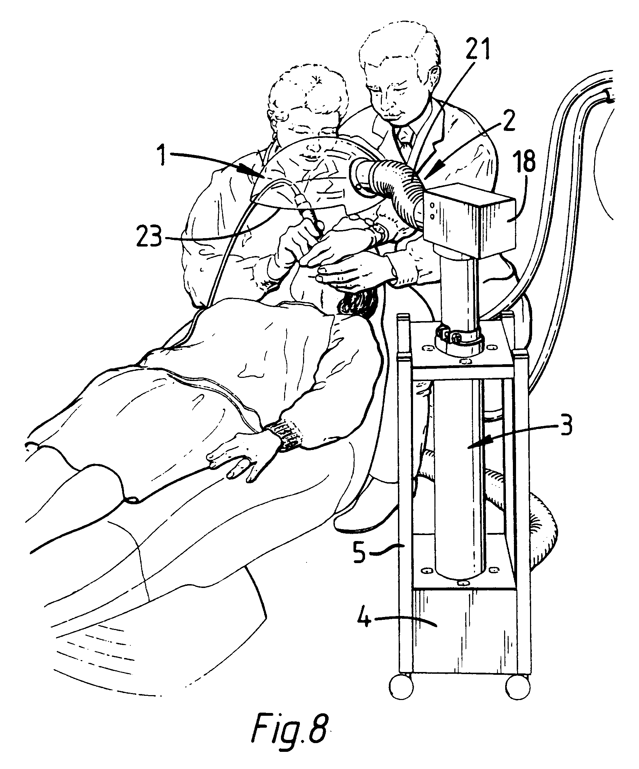

The column 3 is telescopic, allowing for variation of its length using a clamp control 22 (or alternatively, an internal brake device). This enables the height of the arm 2 to be adjusted to whatever is most convenient for optimum location of the hood 1 during use. The manner in which the equipment may be used during the course of dental surgery is illustrated in Figure 8, this showing the surgeon and anaesthetist located near the patient's head and shielded by the hood 1. Gas leaking from the mask used to administer the anaesthetic and mixed with gas and carbon dioxide exhaled by the patient, is drawn up into the hood 1 by the pump or fan unit 4, to be carried away via the tubing 21 and column 3 and thence for safe disposal into the atmosphere via the ducting 6.

The hood 1 can be located close to the patient and this enables high efficiency of gas extraction to be achieved without the necessity for high air-movement and the distracting noise this normally creates. Although most of the gas-contaminated air and carbon dioxide is drawn off directly through the large-bore port 7, the domed configuration of the hood 1 provides a substantial reservoir-volume over the patient's head into which such gases can accumulate temporarily during the drawing-off process. This enables the equipment to keep undesired spillage of gasses outwardly from beneath the hood 1 to a minimum, notwithstanding the normal pulsating breathing pattern of the patient.

The surgeon and the anaesthetist both have an unobstructed view of the patient from virtually all angles, through the transparent hood 1; the spherical dome surface and the low location of the port 7 in the hood 1, ensure that distraction (in spite of any reflection in the hood-surface) and interruption of view are kept to a minimum. They also have ready access under the hood 1 for carrying out the necessary surgical and other procedures. In the latter respect, access under the hood 1 can be increased where desired by a modification shown adopted in Figure 8 and involving relief 23 of the bottom margin of the hood 1 forwardly from its coupling to the arm 2. This modification of the hood 1 is also illustrated in Figures 3 and 4 where the portion cut away for the relief 23 is delineated in broken line.

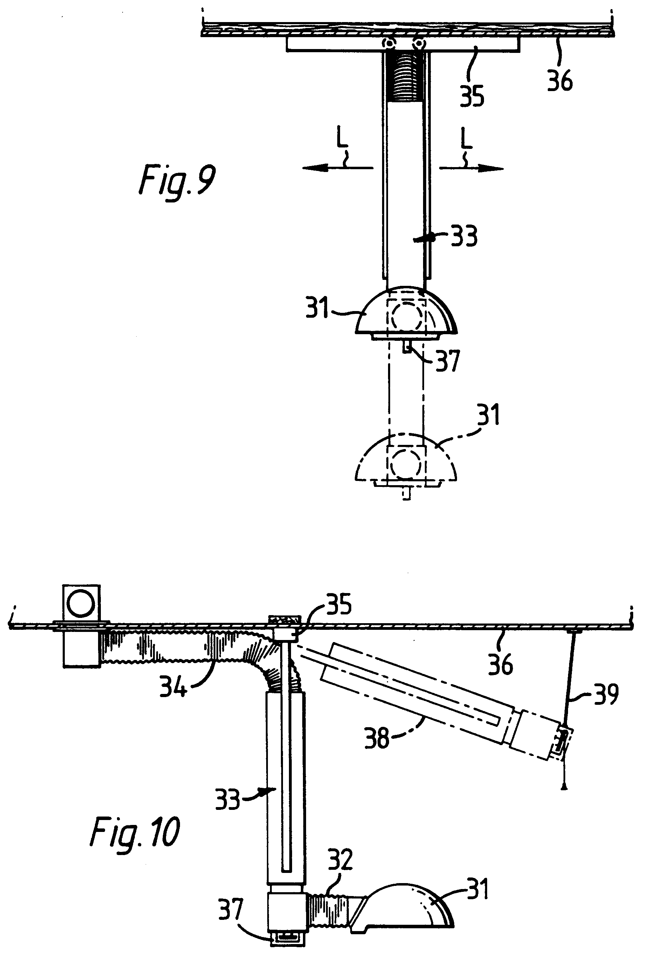

Once the trolley 5 has been stationed, slight pressure by hand on the hood 1 is all that is necessary to adjust the location and orientation of the hood 1 to the best advantage to shield those in attendance whilst allowing them clear access to the patient. The hood 1 can be rapidly swung out of the way simply by pushing it away by hand in the event of emergency or other circumstances calling for its removal. Where appropriate, the equipment rather than being trolley-mounted, may be provided as a fixture, for example mounted on an adjustable spring-balanced arm or on a track. Equipment installed in this latter way, for example, in a dental surgery, is shown in Figures 9 and 10.

Referring to Figures 9 and 10, the hood 31 in this case is carried by an arm 32 that is coupled via a ceiling-mounted telescopic column 33 to exhaust ducting 34. The hood 31 is adjustable in both rotation and tilt with respect to the arm 32, and the column 33 is mounted for movement along a track 35 that is secured to the surgery ceiling 36. The track-mounting of the column 33 allows its position and that of the hood 31 to be adjusted laterally (as indicated by the arrows L in Figure 9). The length of the column 33 is adjustable (indicated in broken line in Figure 9) to allow for variation in height of the hood 31.

The column 33 contains a brake mechanism (not shown) which is normally engaged to restrain change of column-length. A release-control 37 of the brake mechanism is located at the bottom of the column 33, and is operative when gripped by hand to allow the column 33 to be extended or contracted in length simply by pulling it down or pushing it up. The telescopic movement of the column 33 is balanced using a constant tension spring (not shown) so that adjustment of column-length requires only a light pressure up or down on the gripped control 37. Once the length of the column 33 has been adjusted as desired, release of grip on the control 37 re-engages the brake mechanism to hold the column 33 at that length.

As indicated generally by broken line 38 in Figure 10, the column 33 and arm 32 (with the hood 31 removed when not in use) may be stowed out of the way simply by swinging them upwardly for attachment to a ceiling-fitting 39.

A substantial part of the plant used and costs incurred for air-conditioning and ventilating surgical-operating rooms, conventionally relate to the removal of waste anaesthetic gas, required to maintain a safe atmosphere within those rooms. It has been found that savings in plant and costs can be made when equipment of the present invention is utilised, and that safety can be greatly improved. More especially, measurements taken when using equipment constructed as shown in the drawings in normal operation procedures involving nitrous oxide, have shown time-weighted average values for the nitrous oxide in the ambient atmosphere at the attendants' breathing level, of less than 50 parts per million; without the benefit of the gas-extraction equipment the corresponding values are of the order of 2,000 parts per million.

Although the invention has been described above primarily in the context of anaesthesia, it may be applied more generally. In particular, it may find application, for example, in pathology to shield the pathologist not only from gases and vapours hazardous to health but also from unpleasant odours; it may be applied in the latter respect also, in a veterinary context.

## Claims

1. Gas-extraction equipment in which collection of gas to be extracted is via an open mouth (6') of a transparent hood (1;31) that extends from a support arm (2;32), the collected gas is drawn off from the hood (1;31) via a port (7) in the hood (1;31), and the hood (1;31) is adjustable in orientation with respect to the arm (2;32), characterised in that the hood is a transparent shell (1;31) that is substantially hemispherically domed, that the port (7) is located low down on the domed shell (1;31) adjacent the open mouth (6'), and that the shell (1;31) is mounted on the arm (2;32) at the location of the port (7) such that except at that location, view through the hood (1;31) into the region encompassed by the open mouth (6') is substantially unobstructed and undistorted from all angles over and around the domed shell (1;31).

2. Gas-extraction equipment according to Claim 1 wherein the shell (1;31) has a flattened portion surrounding the port (7), and wherein a flange (8) for mounting the hood (1;31) to the arm (2;32) encircles the port (7) within the flattened portion.

3. Gas-extraction equipment according to Claim 1 or Claim 2 wherein the mounting of the shell (1;31) to the arm (2;32) involves a joint (10) located adjacent the shell (1;31) for enabling the hood (1;31) to be adjusted in tilt (T) about an axis transverse to the arm (2;32) and in rotation (R) about a longitudinal axis of the arm (2;32).

4. Gas-extraction equipment according to Claim 3 wherein the joint (10) is balanced such that the hood (1;31) remains in the orientation to which it is adjusted and light hand-pressure is all that is required for adjustment of its orientation.

5. Gas-extraction equipment according to any one of Claims 1 to 4 wherein the path for gas extracted from the hood (1;31) via the port (7), extends internally of the arm (2;32).

6. Gas-extraction equipment according to any one of Claims 1 to 5 wherein the arm (2;32) is carried by a telescopic column (3;33) for height adjustment of the hood (1;31).

7. Gas-extraction equipment according to Claim 6 wherein the column (3) is trolley-mounted (5).

8. Gas-extraction equipment according to Claim 6 wherein the column (33) is mounted on a track (35) for movement laterally (L) of the column (33).

9. Gas-extraction equipment according to Claim 8 wherein the track (35) is ceiling mounted.

10. Gas-extraction equipment according to any one of Claims 1 to 9 wherein the rim of the shell (1) around the open mouth (6') is relieved (23) throughout a substantial part of its periphery away from the port (7).

11. A method of gas-extraction in which the collection of gas to be extracted is via an open mouth (6') of a transparent hood (1;31) that extends from a support arm (2;32), the collected gas is drawn off from the hood (1;31) via a port (7) in the hood (1;31), and the hood (1;31) is adjustable in orientation with respect to the arm (2;32), characterised in that the hood is a transparent shell (1;31) that is substantially hemispherically domed, the port (7) is located low down on the domed shell (1;31) adjacent the open mouth (6'), and the shell (1;31) is mounted on the arm (2;32) at the location of the port (7) such that except at that location, view through the hood (1;31) into the region encompassed by the open mouth (6') is substantially unobstructed and undistorted from all angles over and around the domed shell (1;31).

## Patentansprüche

1. Vorrichtung zum Extrahieren von Gas, wobei das zu extrahierende Gas über einen offenen Mund (6') einer sich von einem Haltearm (2; 32) erstreckenden transparenten Haube (1; 31) aufgefangen wird, das aufgefangene Gas von der Haube (1; 31) über eine Öffnung (7) in der Haube (1; 31) abgezogen wird und die Orientierung der Haube (1; 31) bezüglich des Arms (2; 32) einstellbar ist, **dadurch gekennzeichnet, daß** die Haube eine im wesentlichen halbkugelförmig gewölbte transparente Schale (1; 31) ist, die Öffnung (7) im unteren Abschnitt der gewölbten Schale (1; 31) in der Nähe des Mundes (6') angeordnet ist und die Schale (1; 31) an der Position der Öffnung (7) am Arm (2; 32) angeordnet ist, so daß, mit Ausnahme dieser Position, die Sicht durch die Haube (1; 31) in den durch den Mund (6') umfaßten Bereich von allen Winkeln über und um die gewölbte Schale (1; 31) im wesentlichen nicht behindert oder gestört ist.

2. Vorrichtung zum Extrahieren von Gas nach Anspruch 1, wobei die Schale (1; 31) einen die Öffnung (7) umgebenden abgeflachten Abschnitt aufweist und ein Flansch (8) zum Anordnen der Haube (1; 31) am Arm (2; 32) die Öffnung (7) innerhalb des abgeflachten Abschnitts umgibt.

3. Vorrichtung zum Extrahieren von Gas nach Anspruch 1 oder 2, wobei zum Anbringen der Schale (1; 31) am Arm (2; 32) ein in der Nähe der Schale (1; 31) angeordnetes Gelenk (10) vorgesehen ist, um zu ermöglichen, daß die Haube (1; 31) unter einem Kippwinkel (T) um eine in Querrichtung des Arms (2; 32) ausgerichtete Achse und unter einem Drehwinkel (R) um eine Längsachse des Arms (2, 32) eingestellt werden kann.

4. Vorrichtung zum Extrahieren von Gas nach Anspruch 3, wobei das Gelenk (10) so im Gleichgewicht gehalten wird, daß die Haube (1; 31) in der Orientierung eingestellt bleibt, in der sie eingestellt wurde, und lediglich ein leichter Handdruck erforderlich ist, um ihre Orientierung einzustellen.

5. Vorrichtung zum Extrahieren von Gas nach einem der Ansprüche 1 bis 4, wobei der Weg für das von der Haube (1; 31) über die Öffnung (7) zu extrahierende Gas sich im Arm (2; 32) erstreckt.

6. Vorrichtung zum Extrahieren von Gas nach einem der Ansprüche 1 bis 5, wobei der Arm (2; 32) durch einen Teleskopständer (3; 33) für eine Höheneinstellung der Haube (1; 31) gehalten wird.

7. Vorrichtung zum Extrahieren von Gas nach Anspruch 6, wobei der Ständer (3) auf einem Wagen angeordnet ist (5).

8. Vorrichtung zum Extrahieren von Gas nach Anspruch 6, wobei der Ständer (33) für eine Bewegung in Seitenrichtung (L) des Ständers (33) auf einer Schiene (35) angeordnet ist.

9. Vorrichtung zum Extrahieren von Gas nach Anspruch 8, wobei die Schiene (35) an einer Decke angeordnet ist.

10. Vorrichtung zum Extrahieren von Gas nach einem der Ansprüche 1 bis 9, wobei der Rand der Schale (1) um den Mund (6') über einen wesentlichen Abschnitt seines Umfangs von der Öffnung (7) weg ausgespart ist.

11. Verfahren zum Extrahieren von Gas, wobei das zu extrahierende Gas über einen offenen Mund (6') einer sich von einem Haltearm (2; 32) erstreckenden transparenten Haube (1; 31) aufgefangen wird, das aufgefangene Gas von der Haube (1; 31) über eine Öffnung (7) in der Haube (1; 31) abgezogen wird und die Orientierung der Haube (1; 31) bezüglich des Arms (2; 32) einstellbar ist, **dadurch gekennzeichnet, daß** die Haube eine im wesentlichen halbkugelförmig gewölbte transparente Schale (1; 31) ist, die Öffnung (7) im unteren Abschnitt der gewölbten Schale (1; 31) in der Nähe des Mundes (6') angeordnet ist und die Schale (1; 31) an der Position der Öffnung (7) am Arm (2; 32) angeordnet ist, so daß, mit Ausnahme dieser Position, die Sicht durch die Haube (1; 31) in den durch den Mund (6') umfaßten Bereich von allen Winkeln über und um die gewölbte Schale (1; 31) im wesentlichen nicht behindert oder gestört ist.

## Revendications

1. Equipement d'extraction de gaz dans lequel le gaz à extraire est recueilli par l'intermédiaire d'une ouverture dégagée (6') d'une cloche transparente (1 ; 31) qui s'étend d'un bras support (2 ; 32), le gaz recueilli est aspiré de la cloche (1 ; 31) par l'intermédiaire d'un orifice (7) dans la cloche (1 ; 31) et l'orientation de la cloche (1 ; 31) par rapport au bras (2 ; 32) est réglable, caractérisé en ce que la cloche est une coque transparente (1 ; 31) qui a sensiblement la forme d'un dôme hémisphérique, que l'orifice (7) est situé vers le bas sur la coque bombée (1 ; 31), adjacent à l'ouverture dégagée (6'), et que la coque (1 ; 31) est montée sur le bras (2 ; 32) au niveau de l'emplacement de l'orifice (7) de telle sorte que, sauf au niveau de cet emplacement, la vue à travers la cloche (1 ; 31) dans la région délimitée par l'ouverture dégagée (6') se fait pratiquement sans obstacle, ni déformation, à partir de toutes les directions au-dessus et autour de la coque bombée (1 ; 31).

2. Equipement d'extraction de gaz selon la revendication 1, dans lequel la coque (1 ; 31) a une portion aplatie entourant l'orifice (7) et dans lequel une collerette (8) pour monter la cloche (1 ; 31) sur le bras (2 ; 32) entoure l'orifice (7) à l'intérieur de la portion aplatie.

3. Equipement d'extraction de gaz selon la revendication 1 ou la revendication 2, dans lequel le montage de la coque (1 ; 31) sur le bras (2 ; 32) implique un joint (10) situé adjacent à la coque (1 ; 31) pour permettre de régler l'inclinaison (T) de la coque (1 ; 31) autour d'un axe transversal par rapport au bras (2 ; 32) et sa position angulaire (R) en rotation autour de l'axe longitudinal du bras (2 ; 32).

4. Equipement d'extraction de gaz selon la revendication 3, dans lequel le joint (10) est équilibré de telle sorte que la cloche (1 ; 31) reste dans l'orientation dans laquelle elle a été réglée et qu'une légère pression de la main est tout ce qui est exigé pour régler son orientation.

5. Equipement d'extraction de gaz selon l'une quelconque des revendications 1 à 4, dans lequel le trajet pour le gaz extrait de la cloche (1 ; 31) par l'intermédiaire de l'orifice (7) s'étend à l'intérieur du bras (2 ; 32).

6. Equipement d'extraction de gaz selon l'une quelconque des revendications 1 à 5, dans lequel le bras (2 ; 32) est porté par une colonne télescopique (3 ; 33) pour le réglage en hauteur de la cloche (1 ; 31).

7. Equipement d'extraction de gaz selon la revendication 6, dans lequel la colonne (3) est montée sur un chariot (5).

8. Equipement d'extraction de gaz selon la revendication 6, dans lequel la colonne (33) est montée sur une voie (35) pour déplacer latéralement (L) la colonne (33).

9. Equipement d'extraction de gaz selon la revendication 8, dans lequel la voie (35) est montée au plafond.

10. Equipement d'extraction de gaz selon l'une quelconque des revendications 1 à 9, dans lequel le bord de la coque (1) autour de l'ouverture dégagée (6') est évidé (33) sur une partie importante de sa périphérie en s'éloignant de l'orifice (7).

11. Procédé d'extraction de gaz, dans lequel le gaz a extraire est recueilli par l'intermédiaire d'une ouverture dégagée (6') d'une cloche transparente (1 ; 31) qui s'étend d'un bras support (2 ; 32), le gaz recueilli est aspiré de la cloche (1 ; 31) par l'intermédiaire d'un orifice (7) dans la cloche (1 ; 31), et l'orientation de la cloche (1 ; 31) par rapport au bras (2 ; 32) est réglable, caractérisé en ce que la cloche est une coque transparente (1 ; 31) qui a sensiblement la forme d'un dôme hémisphérique, que l'orifice (7) est situé vers le bas sur la coque bombée (1 ; 31), adjacent à l'ouverture dégagée (6'), et que la coque (1 ; 31) est montée sur le bras (2 ; 32) au niveau de l'emplacement de l'orifice (7) de telle sorte que, sauf au niveau de cet emplacement, la vue à travers la cloche (1 ; 31) dans la région délimitée par l'ouverture dégagée (6') se fait pratiquement sans obstacle, ni déformation à partir de toutes les directions au-dessus et autour de la coque bombée (1 ; 31).
